Europäisches Patentamt

European Patent Office

Office européen des brevets

(19)

(11) Publication number: 0 364 953
A2

# EUROPEAN PATENT APPLICATION

(21) Application number: 89119258.5

(22) Date of filing: 17.10.89

(51) Int. Cl.5: C12Q 1/68 , C07H 21/00 , //C12N15/10

(30) Priority: 18.10.88 JP 260590/88

(43) Date of publication of application:
25.04.90 Bulletin 90/17

(84) Designated Contracting States:
BE CH DE FR GB LI NL SE

(71) Applicant: TEIJIN LIMITED
6-7, Minamihonmachi 1-chome Chuo-ku
Osaka-shi Osaka 541(JP)

(72) Inventor: Nakamura, Yuichi
12-11, Toyooka-cho Tsurumi-ku
Yokohama-shi Kanagawa-ken(JP)
Inventor: Miura, Osamu
2-26-7-703, Sendagi
Bunkyo-ku Tokyo(JP)
Inventor: Aoki, Nobuo
4-20-2-304, Hongou
Bunkyo-ku Tokyo(JP)
Inventor: Sumi, Yoshihiko
3-18-4-113, Tamadaira
Hino-shi Tokyo(JP)

(74) Representative: Kraus, Walter, Dr. et al
Patentanwälte Kraus, Weisert & Partner
Thomas-Wimmer-Ring 15
D-8000 München 22(DE)

(54) Probe for detecting chromosomal aberration induced by chromosomal translocation and method of detecting it.

(57) A probe for detecting a chromosomal aberration induced by chromosomal translocation, said probe comprising a DNA fragment or an RNA fragment containing 50 to 3,000 bases and having the ability to hybridize in a 2 x SSC solution at 40 °C with a DNA sequence existing between the second and third Hind III recognition sites on the bcr gene of a normal human chromosome 22, the Hind III sites being counted from the 3'-side of the first intron of the bcr gene, and a method of detecting a chromosomal aberration in leucocytes and/or lymphocytes, which comprises digesting a genome DNA of leucocytes and/or lymphocytes taken from a subject with a restriction endonuclease, subjecting the resulting DNA fragment to Southern hybridization using the aforesaid probe, and then detecting the resulting band.

## FIELD OF THE INVENTION

This invention relates to a probe for use in detecting a genetic aberration attributed to chromosomal translocation, and to a method of detecting it.

## BACKGROUIND OF THE INVENTION

A chromosomal aberration, particularly the translocation of a chromosomal gene, is known to have closely to do with human cancer. It is known that in chronic myelogenous leukemia (CML) a Philadelphia chromosome formed as a result of translocation of the 9th chromosome to the 22nd chromosome exists [Rowley, J. D.: Nature, 243, 290 (1973)]. In a normal cell, an oncogenic gene c-abl exists on the 9th chromosome. Sometimes, by mutation, for example, the c-abl gene breaks and its fragment is linked to a fragment resulting from breaking of the 22nd chromosome in the region called BCR (break-point cluster region). Consequently, translocation occurs to give a bcr-abl gene. This bcr-abl gene synthesizes mRNA which is different from one synthesized by a normal bcr gene. This mRNA is known to produce a protein having tyrosin kinase activity. This means that an oncogenic DNA is formed as a result of the translocation of the oncogenic gene c-abl on the 9th chromosome to the 22nd chromosome BCR. This is believed to induce CML [see de Klein, A. et al.: Nature, 300, 765 (1982); and Groffen, J., et al.: Cell, 36, 93 (1984)].

cDNA derived from the aforesaid bcr-abl was isolated and a detailed study of chromosomal translocation was undertaken [Hariharan, I. K. and Adams, J. M., EMBO J., 6, 115-119(1L987)].

It was recently reported that acute lymphoblastic leukemia (ALL), in some cases, is induced by the translocation between a human chromosome 9 and a human chromosome 22, and the position of the translocation differs from that in the case of CML [see Hermans, A. et al.: Cell, 51, 33-40 (1987) and Rubin, C.M. et al.: Proc. Natl. Acad. Sci., U. S. A., 85, 2795-2799 (1988)].

In order to detect an aberration of chromosome 22 induced by translocation between chromosome 9 and chromosome 22 in an ALL patient, we prepared a detection probe on the basis of the disclosure of the A. Hermans et al. paper and attempted to detect it. However, we failed to detect the genetic aberration between the second Hind III recognition site from the 3'-side of the first intron of a BCR gene of normal human chromosome 22 and the first Eco RI site from the 3'-side in a domain downstream of the above second Hind III site, which genetic aberration is reported by A. Hermans et al. in the above-cited paper.

We thought that there would be a possibility of the occurrence of a genetic aberration at sites other than those which they reported. Thus, to check such an aberration in a region upstream of the bcr gene of chromosome 22, we obtained a Hind III fragment upstream of the second Hind III site counted from the 3' side of the first intron of BCR of nomrl human chromosome 22, and by using a portion of this fragment as a probe, attempted to detect a chromosomal aberration in ALL patients. This finally led to the determination that a considerable percentage of the patients examined show a genetic aberration in the above-described region of chromosome 22.

Accordingly, we understand that some group of ALL patients show the occurrence of a genetic aberration at sites other than those reported by A. Hermans et al. on BCR of chromosome 22 as a result of chromosomal translocation.

It is an object of this invention therefore to provide a probe for detecting an aberration in chromosome 22 attributed to translocation.

Another object of this invention is to provide a method of detecting a chromosomal aberration in an assay sample, particularly human lymphocytes, by using the probe.

Other objects of this invention along with its features and advantages will become apparent from the following description.

## SUMMARY OF THE INVENTION

According to one aspect of this invention, there is provided a probe for detecting a chromosomal aberration induced by chromosomal translocation, said probe comprising a DNA fragment or an RNA fragment containing 50 to 3,000 bases and having the ability to hybridize in a 2 x SSC solution (17.53 g NaCl, 8.82 g sodium citrate in 1ℓ, adjusted pH 7.0 by NaOH) at 40 °C with a DNA sequence existing between the second and third Hind III recognition sites on the bcr gene of a normal human chromosome 22, the Hind III sites being counted from the 3'-side of the first intron of the bcr gene.

According to another aspect of this invention, there is method of detecting a chromosomal aberration in lymphocytes, which comprises digesting a genome DNA of lymphocytes taken from a subject with a restriction endonuclease, subjecting the resulting DNA fragment to Southern hybridization using the above probe, and then detecting the band(s) of the DNA fragment(s).

## BRIEF DESCRIPTION OF THE DRAWINGS

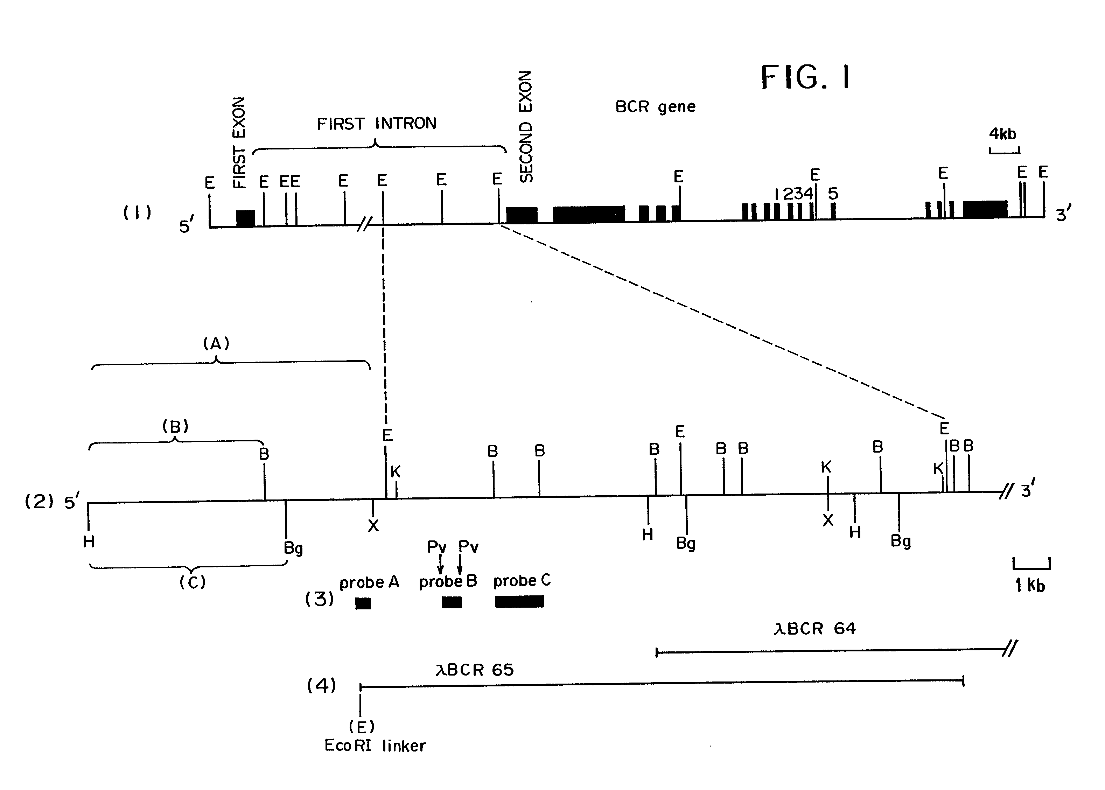

Figure 1, (1) is a restriction endonucleasee map of bcr gene on chromosome 22, in which black rectangles show exons, fine lines show introns, and E shows restricion endocnuclease Eco RI.

Figure 1, (2) is a restriction endocuclease map of part of the first intron of the BCR gene ranging from the third Hind III cleavage site from the 3′-side of the first intron in Figure 1, (1) to a position 1.5kb downstream toward the 3′-side from the first Eco RI site from the 3′-side, in which B, Bg, E, H, K and X respectively stand for restriction endonucleases Bam HI, Bgl II, Eco RI, Hind III, Kpn I and Xho I. It is uncertain whether there are Eco RI, Kpn I and Xho I sites in the part (A). It is uncertain whether there is a Bam HI site in the part (B). It is uncertain whether there is a Bgl II site in the part (C).

Figure 1, (3) shows the positions of probes used in the examples, in which probe A is a 0.15 kb fragment upstream of the Xho I site on the 5′-side of Figure 1, (2); probe B is a 0.5 kb fragment obtained by digesting a DNA fragment interposed between the Kpn I site and the Bam HI site on the 5′-side in Figure 1, (2) with restriction endonuclease Pvu II; and probe C is a 1.3 kb fragment obtained by digestion with the first Bam HI and the second Bam HI counted from the 5′-side in Figure 1, (2).

Figure 1, (4) shows DNA fragments in a phage clone which encode the human bcr gene in accordance with this invention.

Figures 1, (2) to (4) correspond to one another vertically.

## DETAILED DESCRIPTION OF THE INVENTION

As stated above, we found that some group of ALL patients have a genetic aberration attributed to translocation between the 2nd and 3rd Hind III sites from the 3′-side to the 5′-side of the first intron on bcr gene of human chromosome 22.

After chromosomal translocation, the restriction endonuclease map of a chromosomal DNA in the neighborhood of the site of translocation changes, and the sizes of DNA fragments resulting from digestion of the chromosomal DNA with restriction endonucleases change from those in a normal condition. Accordingly, when a genome DNA of an ALL patient and a genome DNA of a normal human are digested with a suitable restriction endonuclease, and then subjected to Southern hybridization by using a DNA fragment near the site of translocation as a probe, a band of a size not seen in the case of a sample from a normal human is observed in the sample from the ALL patient. Consequently, the occurrence of translocation can be detected.

The differences between DNA fragments of a normal human and those of an ALL patient are shown in Table 1 when they are prepared by using typical restriction endonucleases Eco RI, Hind III, Bam HI and Bgl II, and subjected to Southern hybridization.

Table 1

| Restriction endonuclease | Normal control | | | ALL patient | | (Unit: kb) |
|---|---|---|---|---|---|---|
| | X | Y | Z | 1 | 2 | |
| Eco RI | 10.3<br>8.5 | 10.3 | 8.5 | 9.7<br>8.5 | 8.5<br>7 | |
| Hind III | 13.5<br>12.5 | 12.5 | 13.5 | 13.5<br>8.5 | 13.5<br>10 | |
| Bam HI | 5.5<br>4.4 | 4.4 | 5.5 | 10<br>5.5 | 5.5 | |
| Bgl II | 9.6<br>8.5 | 8.5 | 9.6 | 12<br>9.6 | 12<br>9.6 | |

In the above examples, the DNA fragments are prepared by using the specific restriction endonucleases. Even when a different restriction endonuclease is used DNA fragments of different lengths are obtained from the normal control and the ALL patient although the length of each DNA fragment is different from that obtained by using the specific restriction endonucleases. Accordingly, the difference can also be detected by using the probe of this invention.

The differences are also noted among samples from normal humans X, Y and Z. This is presumably due to polymorphism in intron I [see Rubin, C. M. et al.: Nucleic Acids Research, 16 (17), 8741 (1988)]. The difference, however, does not affect the identification of ALL patients.

The preparation of the probe of this invention will be described below in detail.

A general method of preparing a probe will first be described. Cells are taken out from a suitable site of a normal human, and DNA is extracted and purified. The DNA is then digested partially with a suitable restriction endonuclease such as Sau IIIA. From the resulting DNA fragment, a gene library is constructed by using a phage vector such as λgtwes λB, Charon 3A, Charon 4A, Charon 17, Charon 20, Charon 21A and Charon 28 or a plasmid vector such as pJB8, MUA-3 and Kos 1.

Then, a DNA base sequence on the 5'-side of the second exon of bcr gene, for example a DNA sequence having a size of 70 bp, and a base sequence coomplementary to it are synthesized.

BCR 1. 5'ATGGCTCGTTCGGAACACCACCTGGATACGG

CTGCGCTGCAGACCGGGCAGAGGAGCAGCGCC

GGCACCAA 3'

BCR 2. 5'TTGGTGCCGGCGCTGCTCCTCTGCCCGGTCT

GCAGCGCAGCCGTATCCAGGTGGTGTTCCGAA

CGAGCCAT 3'

By screening the gene library using these base sequences as probes, DNA near the second exon of the bcr gene can be obtained. The resulting DNA is digested with various restriction endonucleases such as Eco RI, Bam HI, Hind III and Kpn I, and a restriction endonuclease map is prepared. A clone having three Hind III cleavage sites upstream (5'-side) of the second exon of the bcr gene is selected. Digestion of the resulting bcr gene fragment with Hind III can give a DNA fragment having a size of 12 to 14 kb. Sometimes, two DNA bands are observed between them. This is due to polymorphism, and both can be used for the preparation of the probe of this invention.

This DNA fragment corresponds to a DNA sequence existing between the second Hind III site and the third Hind III site from the 3'-side of the first intron of the bcr gene on a human normal chromosome 22 as referred to in the present invention.

The probe of this invention can be prepared firstly by digesting the above DNA fragment with a suitable restriction endonuclease on the basis of the restriction endnuclease map of Figure 1. Secondly, it can be obtained by sequencing the base sequence of the above DNA fragment, and chemically synthesizing part of it. At this time, non-complementary base may be replaced or mixed in an amount which does not impair the hybridizing ability specified in this inventionn, for example, in an amount of not more than 10 % of the entire probe. Thirdly, a corresponding RNA fragment of partial RNA fragment is prepared from the above DNA fragment or its partial DNA fragment by using a bacteriophage SP6 promoter in accordance with the method of Melton et al. [D. A. Melton et al.: Nucleic Acid Research, 12 (18), 7035 -7056 (1984)], and used as the probe of this invention.

The probe of this invention should have the ability to hybridize with a DNA sequence existing between the second and third Hind III sites from the 3'-side of the first intron of the bcr gene on a human normal chromosome 22. Whether or not it has the hybridizing ability can be determined in accordance with the method of Southern 5 [see Southern E., J. Mol. Biol. 98, 503 (1975)]. Specifically, the DNA sequence existing between the second and third Hind III sites upstream of the 3'-terminus of the first intron of the bcr gene in normal human chromosome 22 is fixed to a nitrocellulose filter, and whether the probe, labelled, binds to the DNA fragment fixed to the nitrocellulose, in a 2 x SSC solution at 40 °C is determined.

Conveniently, the DNA fragment or RNA fragment used as the probe of this invention contains 50 to 3,000, preferably 100 to 2,000, especially preferably 400 to 1,000, bases. So long as the DNA or RNA fragment used as the probe of this invention has the ability to hybridize with the DNA sequence existing between the second and third Hind III sites upstream of the 3'-terminus of the first intron of the bcr gene on normal human chromosome 22, it does not have to be completely complementary to the above DNA sequence, btu desirably, it contains a base sequence substantially complementary to a corresponding portion having 50 to 3,000 bases.

The DNA or RNA fragment constituting the probe of this invention can be advantageously derived from leucocyte and/or lymphocytes in normal human peripheral blood or from a normal human placenta cell.

For use as the probe of this invention, the above DNA or RNA fragment is labelled in a customary manner. The labelling can be effected by binding a labelling material, for example, a radioisotope, a fluorescent substance, a phosphorescent substance, an enzyme, biotin or avidin to the DNA or RNA fragment by methods known per se [see, for example, Rigby P. W. et al., J. Mol. Biol. 113, 237-251 (1977), Maxam A.M. et al, Methods Enzymol. 65, 499-560 (1980), Leary J.J., Proc, Natl. Acad. Sci. USA 80, 4045-4049 (1983), Forster A. C. et al., Nucl. Acids Res, 13, 745-761 (1985), Sheldon E.L. et al., Proc. Natl. Acad. Sci. USA 83, 9085-9089 (1986), Renz M. et al., Nucl. Acids Res. 12, 3435-3444 (1984), Jablonski E. et al., Nucl. Acids Res. 14, 6115-6128 (1986), and Kempe J. et al., Nucl. Acids Res. 13, 45-57 (1985) for the method of labelling]. Suitabel labelling materials include radioisotopes such as $^{125}$I, $^{131}$I, $^{32}$P, $^{35}$S and $^{3}$H; Fluorescent substances such as N-acetoxy-N2-acetylamino-fluorene (AFF) and N-acetoxy-N2-acetylamino-7-iodofluorene (AAIF) and phosphorescent substances such as photobiotin.

The probe of this invention can be effectively used in detecting a genetic aberration by translocation which occurs in an assay sample, for example, between the second and third Hind III recognition sites on the bcr gene of chromosome 22 of an ALL patient counted upstream from the 3'-side of the bcr gene. The detection may be performed by, for example, digesting a genome DNA of lymphocytes from the subject with a restriction enzyme, subjecting the DNA fragment to Southern hybridization [see Southern E., J. Mol. Biol. 98 503 (1975)] and thereafter, detecting the band(s) of DNA fragment(s).

More specifically, the detection can be performed by the followong procedure.

Leucocytes and/or lymphocytes are taken out from an assay sample, for example the peripheral blood of an ALL patient, DNA is extracted from the cells and purified. The purified DNA is digested with a suitable restriction endonuclease, such as Eco RI, Bam HI, Hind III, Bgl II, Kpn I, Sac I, Xho I and Xba I. The DNA fragment is subjected to gel electrophoresis, denatured with alkali, and the DNA is blotted on a nitrocellulose filter or the like. The nitrocellulose filter blotted with the DNA and the labeled probe are hybridized in a 6 x SSC solution (52.59 g NaCl, 26.46 g sodium citrate in 1ℓ, adjusted pH 7.0 by NaOH) at 60 °C for a suitable period of time of, for example, 8 hours. The hybridization product is washed wih a 2 x SSC solution at 50 °C for 1 hour, and then autoradiographed.

Since the probe of this invention has such a high specificity that it hybridizes with the target domain in a 2 x SSC solution at 40 °C, it is non-specific for other domains. Thus, when washing is carried out under the above conditions, all hybridization products formed in the other domains are removed, and a high accuracy of analysis can be obtained.

By the method of detecting a chromosomal aberration of leucocyte and/or lymphocytes using the probe of this invention, a genetic aberration by translocaion within the aforesaid domain on the bcr gene of the human chromosome 22 can be detected effectively. Hence, this method of detection permits prediction and early discovery of ALL considered to be attributed to such a genetic aberration, and the determination of remission after treatment of an ALL patient.

For example, ALL patient-2 shown in Table 1 showed remission after chemotherapy following onset. A genome DNA from the patient after remission is subjected to the same analysis as above using Eco RI as a restriction endonuclease. Only one band of about 8.5 kb is observed. This shows that a leucocytes and/or lymphocytes in which chromosomal translocation occurred has been almost completely removed from the patient after remission. This also demonstrates that the detection probe and method of this invention are accurate.

The following Examples illustrate the isolation of the bcr gene, the preparation of diagnostic probes, the preparation of diagnostic RNA probes, and Southern hybridization.

EXAMPLE 1

Isolation of the bcr gene

Two DNAs (shown hereinabove), one having a DNA base sequence corrsespoding to a size of 70 bp on the 5′-side of the second exon of the bcr gene reported by Hariharan et al. [see Hariharan, I. K. and Adams, J. M.: EMBO J. 6, 115-119 (1987)] and the other having a base sequence complementary to it, were synthesized by a DNA synthesizer (supplied by Applied Biosystems, Inc.). The synthesized DNAs were purified and dissolved each in a concentration of 50 μg/ml in a solution of 10 mM Tris-HCl (pH 7.6) and 1 mM EDTA, heated at 70 °C for 30n minutes in a water vessel, and thereafter allowed to cool spontaneously to room temperature to anneal two synthetic DNAs. These double-stranded DNAs were labelled with $^{32}P$ by nick translation using [$^{32}P$]-dCTP. A chromosomal gene library (about 500, plaques) derived from human placenta was prepared through an Eco RI linker, and then screened by using the above labelled probes. As a result, a clone of λBCR64 was obtained [see Figure 1, (4)]. Furthermore, the above library was screened by using a DNA fragment on the 5′-side of λBCR64 clone to obtain a clone (λBCR65) containing an upstream portion from the 5′-side. These clones were respectively digested with various restricion endonucleases, and a restriction endonuclease map was prepared A gene having a size of about 7.5 from the 5′-terminus of λBCRF65 to the Hind III cleavage site counted from that terminus has not been published in the literature, and can thus be consdered as a novel DNA fragment.

EXAMPLE 2

Preparation of diagnostic probes

The insert DNA of λBCR65 was digested with restriction enzymes Eco RI and Xho I. The resulting DNA fragment having a size of about 0.15 kb was isolated and used as a probe A. Furthermore, λBCR65 was digested with restriction endonuclease Pvu II, and the resuting DNA fragment having a size of about 0.5 kb was isolated, and used as a probe B. Furthermore, λBCR65 was digested with restriction endonnuclease Bam HI. The resulting DNA fragment having a size of about 1.3 kb was isolated and used as probe C.

The probes were each labelled by nick translation using α[$^{32}P$]-dCTP.

These probes could be hybridized with the DNA fragment of about 7.5 kb in a 2 x SSC solution at 50 °C.

EXAMPLE 3

Preparation of diagnostic RNA probes

Vector pSP72 (Riboprobe Gemini System, a proudct of Promega Company) was digested with restriction endonuclease Bam HI and treated with bovine alkaline phosphatase. The resulting product was ligated with probe C prepared in Example 2 by using a DNA ligase to form plasmid pSP72-PRC. An RNA probe corresponding to the DNA base sequence of probe C was synthesized by using the resulting plasmid in accordance with the method of Melton et al. [see Melton, D. A. et al.: Nuc. Acid Res., 12, 7035-7056 (1984)]. First, PSP72-PRC was digested with restriction endonuclease Sma I, and ATP, GTP, UTP and α-[$^{32}$P]CTP were added. SP6 RNA polymerase (a product of Riboprobe SP RNA Polymerase, a product of Promega Company) was added in the presence of an RNA decomposing enzyme inhibitor (RNasin, a product of Promega Compmany), and the reaction was carried out at 37 °C for 2 hours. After the reaction, DNA decomposing enzyme, DNase I, was added and the reaction was carried out at 37 °C for 10 minutes. Proteins were removed by using a mixture of chloroform and phenol, and then the aqueous layer was subjected to Sephadex G-50 Gel column, and a $^{32}$P-labelled RNA was recovered as a probe.

These probes could be hybridized with the DNA fragment of about 7.5 kb in a 2 x SSC solution at 50 °C.

EXAMPLE 4

Southern hybridization

Isolation of lymphocytes from human peripheral blood, extraction of chromosomal DNA from the lymphocytes and Southern hybridization were carried out in accordance with the method of Drabkin et al. [see Drabkin, H. A., et al.: Proc. Natl. Acad. Sci., U. S. A., 82, 464-468 (1985)].

Chromosomal DNAs extracted from the peripheral blood of normal control and ALL patients, each in an amount of about 5 μg, were each digested completely with the restriction endonucleases indicated in Tables 2 and 3, electrophoresed on a 1.0 % agarose gel, and then transferred to nitrocellulose filters using a 10 x SSC solution (87.65 g NaCl, 44.1 g sodium citrate in 1 ℓ, adjusted pH 7.0 by NaOH). The filters were baked at 80 °C for about 2 hours. Southern hybridization was carried out on these nitrocellulose filters by using probe B or probe C. The filters were washed with a 2 x SSC solution at 50 °C to remove the undesired hybridization products, followed by autoradiography.

When thee genome DNA of a normal human was digested with restriction endonuclease Eco RI and subjected to Southern hybridization using probe B, a single band having a size of about 8.5 kb, a single band having a size of about 10.3 kb, or two bands having a size of about 8.5 kb and 10.3 kb were obtained. This difference is considered to be due to the presence of polymorphism in intron I.

On the other hand, when the above Southern hybridization was carried out on the genome DNA of an ALL patient (ALL-1), two bands having a size of about 9.5 kb and about 9.7 kb were observed. It was shown that the band of about 8.5 kb was derived from the normal genome DNA, but the band of about 9.7 kb was an extra-band resulting from chromosomal translocation. In the case of another ALL patient (ALL-2), two bands of about 8.5 kb and about 7 kb were seen, and the band of about 7 kg is an extra-band attributed to chromosomal translocation. The patient (ALL-2) was treated by chemotherapy after onset, and showed remission. When the same experiment was conducted on the genome DNA of ALL-2 after remission, only a single band of about 8.5 kb was observed. This shows that leucocytes and/or lymphocytes at which chromosomal translocation occurred was removed almost completely.

The above experiment was repeated by using restriction endonucleases Hind III, Bam HI and Bgl II. The results are summarized in Table 2.

The above Sourthern hybridization was carried out by using probe C and restriction endonucleases Eco RI and Bgl II. The patterns of the bands obtained are summarized in Table 3.

7

Table 2

| Restriction endonuclease | Normal control | | | ALL patient | | (Unit: kb) |
|---|---|---|---|---|---|---|
| | X | Y | Z | 1 | 2 | 2* |
| Eco RI | 10.3 8.5 | 10.3 | 8.5 | 9.7 8.5 | 8.5 7 | 8.5 |
| Hind III | 13.5 12.5 | 12.5 | 13.5 | 13.5 8.5 | 13.5 10 | 13.5 |
| Bam HI | 5.5 4.4 | 4.4 | 5.5 | 10 5.5 | 5.5 | 5.5 |
| Bgl II | 9.6 8.5 | 8.5 | 9.6 | 12 9.6 | 12 9.6 | 9.6 |
| ALL patient 2*: after remission | | | | | | |

Table 3

| Restriction endonuclease | Normal control | | | ALL patient | (Unit: kb) |
|---|---|---|---|---|---|
| | X | Y | Z | 2 | |
| Eco RI | 10.3 8.5 | 10.3 | 8.5 | 8.5 7 | |
| Bgl II | 9.6 8.5 | 8.5 | 9.6 | 12 9.6 | |

The above results show that on the 5'-side of the restriction endonuclease map shown in Figure 1, (2), the Bgl II cleavage site exists at about 2.0 kb (about 0.9 kb), the Bam HI cleavage site exists at about 2.5 kb (about 1.4 kb), and the Hind III cleavage site exists at about 6.7 kb (about 5.6 kb) (the parenthesized figures are due to polymorphism) counted from the secound Xho I cleavage site from the 3'-side of the first intron. It is uncertain whether there are Eco RI, Kpn I and Xho I sites in the part (A). It is uncertain whether there is a Bam HI site in the part (B). It is uncertain whether there is a Bgl II site in the part (C). Accordingly, DNA fragments composed of DNA molecules or RNA molecules which hybridize with these portions can also be utilized for the purpose of this invention.

## Claims

1. A probe for detecting a chromosomal aberration induced by chromosomal translocation, said probe comprising a DNA fragment or an RNA fragment containing 50 to 3,000 bases and having the ability to hybridize in a 2 x SSC solution at 40 °C with a DNA sequence existing between the second and third Hind III recognition sites on the bcr gene of a normal human chromosome 22, the Hind III sites being counted from the 3'-side of the first intron of the bcr gene.

2. The probe of claim 1 in which the DNA fragment or the RNA fragment contains a base sequence substantially complementary to that portion of said DNA sequence which contains 50 to 3,000 bases.

3. The probe of claim 1 in which the DNA fragment or the RNA fragment contains 100 to 2,000 bases.

4. The probe of claim 1 which is labelled with a radioisotope, a fluorescent substance or a phosphorescent substance.

5. The probe of claim 1 in which the normal human chromosome 22 is derived from leucocytes and/or lymphocytes isolated from normal human peripheral blood.

6. The probe of claim 1 in which the normal human chromosome 22 is derived from a normal human placenta cell.

7. The probe of claim 1 for use in detecting acute leucoblastic and/or lymphoblastic leukemia.

8. A method of detecting a chromosomal aberration in leucocytes and/or lymphocytes, which comprises digesting a genome DNA of leucocytes and/or lymphocytes taken from a subject with a restriction endonuclease, subjecting the resulting DNA fragment to Southern hybridization using the probe of claim 1, and then detecting the resulting band.

# FIG. 1

FIRST EXON

FIRST INTRON

SECOND EXON

BCR gene

4kb

(1) 5′ ... E ... E E E ... E ... E ... E ... E ... E ... 1 2 3 4 | 5 ... E ... E ... E E ... 3′

(A)

(B)

(2) 5′ ... B ... E ... K ... B ... B ... B E ... B B ... K ... B ... K B B ... // 3′

H ... Bg ... X ... H ... Bg ... X ... H ... Bg

(C)

Pv   Pv

(3)   probe A   probe B   probe C

λBCR 64

λBCR 65

(4)

(E)
EcoRI linker

EP 0 364 953 A2